# EUROPÄISCHE PATENTSCHRIFT

(11) **EP 1 221 902 B1**
(45) Veröffentlichungstag und Bekanntmachung des Hinweises auf die Patenterteilung: **21.12.2005**
(21) Anmeldenummer: 01965172.8
(22) Anmeldetag: 04.08.2001
(51) Int. Cl.: A61B 18/14

(54) **UROLOGISCHES, ELEKTROCHIRURGISCHES RESEKTOSKOP**
UROLOGICAL ELECTROSURGICAL RESECTOSCOPE
RESECTOSCOPE UROLOGIQUE ELECTROCHIRURGIQUE

(30) Priorität: 26.08.2000 DE 10042097
(43) Veröffentlichungstag der Anmeldung: 17.07.2002
(73) Patentinhaber: OLYMPUS WINTER & IBE GMBH, 22045 Hamburg (DE)
(72) Erfinder: BROMMERSMA, Pieter, 22941 Bargteheide (DE); NUSSBAUM, Felix, 22089 Hamburg (DE)
(74) Vertreter: Schaefer, Konrad
(86) Internationale Anmeldenummer: PCT/EP2001/009045
(87) Internationale Veröffentlichungsnummer: WO 2002/017808

(56) Entgegenhaltungen:
- DE-A- 3 917 583
- DE-A- 3 918 316
- FR-A- 2 400 351

## Beschreibung

Die Erfindung betrifft ein Resektoskop der im Oberbegriff des Anspruch 1 genannten Art.

Gattungsgemäße Resektoskope sind in erster Linie für die Prostataresektion vorgesehen, jedoch ihrer Bauart nach gegebenenfalls auch für andere chirurgische Einsätze verwendbar. Unter Resektoskop werden hier endoskopische Geräte verstanden, bei denen in einem Schaftrohr eine Optik und ein Elektrodenträger mit distaler Elektrode angeordnet sind, wobei der Elektrodenträger mit der Elektrode in Achsrichtung verschiebbar gelagert ist und mit seinem proximalen Ende an einem Schiebekörper des Resektoskopes befestigt und elektrisch kontaktiert ist, welcher mit einer Griffbetätigung von Hand axial verstellt werden kann, um die Elektrode axial zu verschieben.

Bei der Prostataresektion wird das Resektoskop mit dem distalen Ende des Schaftrohres durch die Harnröhre bis in das Innere der Prostata vorgeschoben. Bei eingeschalteter HF-Beaufschlagung der Elektrode kann diese durch Handbewegung des Schiebekörpers, an dem sie proximal befestigt ist, vor- und zurückgeschoben werden, um Gewebe zu schneiden. Die Elektrode ist dabei üblicherweise als Drahtschlinge ausgebildet, mit der Gewebeschnipsel resiziert werden können. Die Elektrode kann auch anders ausgebildet sein, beispielsweise als Knopfelektrode, Rollenelektrode, Messerelektrode oder dergleichen, um für unterschiedliche Einsatzzwecke z. B. Koagulieren, Schneiden und dergleichen verwendet werden zu können.

Probleme bildet dabei stets die ordnungsgemäße Kontaktierung des die Elektrode stromversorgenden Leiterdrahtes an seinem Kontaktabschnitt im Schiebekörper. Dort muß der Kontakt mit einem weiterführenden Kabel hergestellt werden, das zu einem getrennt aufgestellten HF-Generator führt. Kontaktstellen mit HF-Beaufschlagung sind problematisch und neigen zu Kontaktfehlern bzw. zum Verschmoren.

Bei älteren Konstruktionen wurde im Schiebekörper mit einer Klemmschraube gleichzeitig kontaktiert und die mechanische Befestigung des Elektrodenträgers sichergestellt. Verschmort diese Kontaktierungsstelle, so muß der gesamte Schiebekörper ausgewechselt werden.

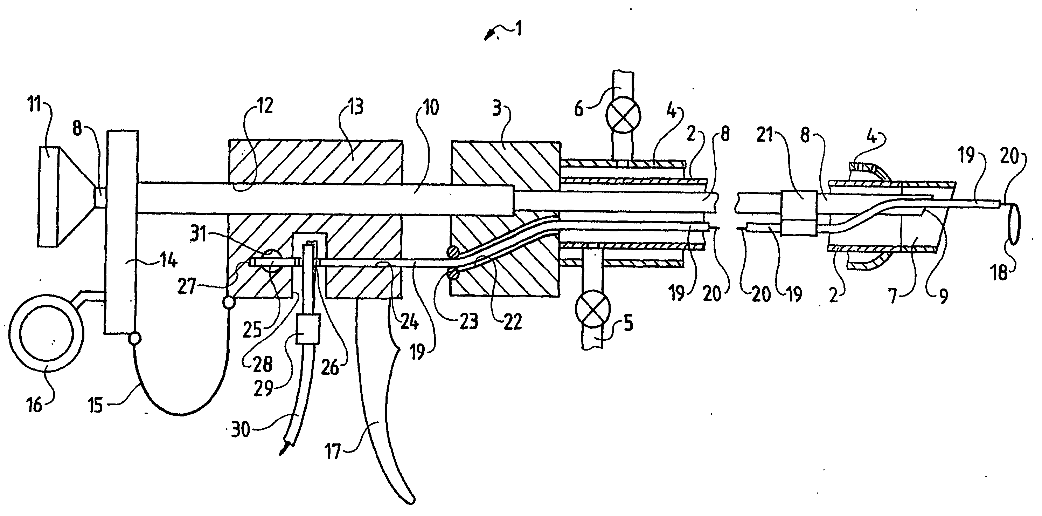

Bei der nicht gattungsgemäßen WO-A-96/23449, Fig. 16 handelt es sich um ein Resektoskop mit bipolarer Elektrode, bei der also der Elektrodenträger von zwei Leiterdrähten durchlaufen wird. Der Elektrodenträger weist im Bereich des Schiebekörpers folglich zwei Kontaktabschnitte auf. Ein auf den Schiebekörper aufsteckbarer Stecker des weiterführenden Kabels kontaktiert beide Kontaktabschnitte, wobei er an beiden Kontaktabschnitten sowohl kontaktiert als auch klemmt. Eine besondere, getrennte Befestigungseinrichtung ist nicht vorgesehen. Es wird also hier stets gleichzeitig kontaktiert und geklemmt. Eine Befestigung des Elektrodenträgers zum Ausprobieren der mechanischen Funktion vor dem Kontaktiervorgang ist nicht möglich.

Eine gattungsgemäße Konstruktion ist aus US 4,917,621 und US 4,919,131, jeweils in Fig. 3 bekannt. Der Schiebekörper weist einen quer durchgehenden Schacht auf, in den der Stecker des weiterführenden HF-Kabels zur Kontaktierung des im Schacht frei liegenden Kontaktabschnitts des Elektrodenträgers einsteckbar ist. Distal vom Schacht ist eine an einem Befestigungsabschnitt des Elektrodenträgers angreifende Klemmeinrichtung vorgesehen.

Vorteilhaft an dieser Konstruktion ist die Möglichkeit, den Elektrodenträger mit der Klemmeinrichtung am Schiebekörper gesondert mechanisch zu befestigen, so daß seine ordnungsgemäße mechanische Funktion zunächst ausprobiert werden kann. Anschließend kann mit dem Stecker kontaktiert werden. Verschmort die Kontaktierungsstelle, so muß nur der Elektrodenträger und das Kabel mit dem Stecker gewechselt werden. Die Klemmeinrichtung und der Schiebekörper bleiben unversehrt, da die Klemmeinrichtung gesondert ausgebildet ist.

### Diese bekannte gattungsgemäße Konstruktion weist jedoch auch Nachteile auf

Da die Befestigungseinrichtung distal von der Kontaktiereinrichtung liegt, wird die Stelle des Elektrodenträgers, an der die Befestigung erfolgt, von dem Leiter durchlaufen, der den Kontaktabschnitt mit der Elektrode verbindet. Folglich ist der Elektrodenträger an dieser Stelle nicht sehr stabil. Die Befestigungseinrichtung muß darauf Rücksicht nehmen und kann beispielsweise nur mit sehr geringen Klemmkräften arbeiten. Erfolgt die Befestigung mit einem in eine Nut auf dem Elektrodenträger eingreifenden Schieber, so kann die Nut nur sehr flach ausgebildet sein, woraus sich eine verringerte Befestigungssicherheit ergibt.

Der proximale Endbereich des Elektrodenträgers wird von dem Bereich gebildet, an dem die Befestigung erfolgt, sowie von dem Kontaktierbereich. Diese Bereiche, also der gesamte Endbereich des Elektrodenträgers sind folglich starr und biegesteifer als der restliche Teil des Elektrodenträgers, der nur aus einem Innenleiter und einer Außenisolierung besteht. Bei Resektoskopen liegt jedoch üblicherweise der Elektrodenträger im Schaftrohr dicht benachbart neben der Optik, während er im Bereich des Schiebekörpers im größeren Abstand zu diesem liegen muß, um Platz zu schaffen für die Kontaktiereinrichtung und die Befestigungseinrichtung. Folglich muß der Elektrodenträger im Hauptkörper verschwenkt geführt werden, wie dies z. B. in der WO-A-96/23 449, Fig. 13 angedeutet ist. Da wiederum der Hauptkörper aus konstruktiven Gründen nicht zu lang sein darf, muß auf kurzem Wege stark verschwenkt werden. Hierbei stört jedoch die erhebliche Länge des starreren Endbereiches des Elektrodenträgers der bekannten Konstruktion.

Ferner sind Fehlmontagen möglich, wenn der Elektrodenträger nicht weit genug eingesteckt und dann befestigt wird.

Die Aufgabe der vorliegenden Erfindung besteht darin, ein gattungsgemäßes Resektoskop zu schaffen, bei dem der Elektrodenträger störungsfrei im Schiebekörper befestigt und kontaktiert werden kann.

Diese Aufgabe wird mit den Merkmalen des Anspruches 1 gelöst.

Erfindungsgemäß ist im Schiebekörper die Befestigungseinrichtung proximal der Kontaktiereinrichtung vorgesehen. Entsprechend ist ein Elektrodenträger erforderlich, bei dem der Befestigungsabschnitt proximal vom Kontaktabschnitt liegt. Folglich muß der Befestigungsabschnitt des Elektrodenträgers nicht mehr von einem Innenleiter durchlaufen werden und kann sehr stabil z. B. als massives Metallstück ausgebildet sein. Es können verschiedene sehr sicher wirkende Befestigungsmethoden verwendet werden, z.B. Klemmung mit hoher Klemmkraft, Verriegelung auf tiefen Nuten oder sogar Verriegelung mit einem Stift durch eine Querbohrung des Elektrodenträgers. Es lassen sich auch gut haltende Rastverbindungen vorsehen. Ein weiterer Vorteil der proximalen Anordnung der Befestigungseinrichtung besteht darin, daß bei ordnungsgemäßer Befestigung sichergestellt ist, daß der Elektrodenträger vollständig eingesteckt ist, also an der Stelle der Kontaktiereinrichtung der Kontaktabschnitt des Elektrodenträgers liegt und somit eine Kontaktierung erfolgen kann. Schließlich läßt sich auch sehr wirkungsvoll das Problem der Verschwenkung des steifen Endabschnittes des Elektrodenträgers beim Einführen durch den Hauptkörper lösen. Das biegesteife Endstück bestehend aus dem Kontaktabschnitt und dem Befestigungsabschnitt läßt sich kürzer ausbilden. Der Befestigungsabschnitt des Elektrodenträgers kann ferner auch mit geringerem Durchmesser ausgebildet werden, was ebenfalls die Durchführung durch eine engere Verschwenkung ermöglicht.

Vorteilhaft sind die Merkmale des Anspruches 2 vorgesehen. Der Endanschlag kann beispielsweise durch das Ende der Aufnahmebohrung im Schiebekörper gebildet sein. Beim Einstecken das Elektrodenträgers kann dieser bis auf Anschlag eingeschoben werden und liegt dann hochgenau mit seinem Befestigungsabschnitt an der Befestigungseinrichtung und mit seinem Kontaktabschnitt an der Kontaktiereinrichtung. Ein Endanschlag kann auch an der Befestigungseinrichtung selbst vorgesehen sein, beispielsweise an einer Klemmschraube, einem Klemmschieber oder einer Rastvorrichtung.

In der Zeichnung ist die Erfindung beispielsweise und schematisch mit einem Achsschnitt eines Resektoskopes mit montierter Elektrode dargestellt.

Das dargestellte Resektoskop 1 weist ein Schaftrohr 2 auf, das mit seinem proximalem Ende an einem Hauptkörper 3 befestigt ist. In nicht dargestellter Weise kann das Schaftrohr 2 mit der üblichen Kupplungseinrichtung abnehmbar am Hauptkörper 3 befestigt sein. Um das Schaftrohr 2 herum ist ein Außenrohr 4 vorgesehen, das ebenfalls am Hauptkörper 3 befestigt ist und zwar ebenfalls in der üblichen Weise mit einer nicht dargestellten Kupplung. Das Innere des Schaftrohres 2 dient in üblicher Weise als Zulaufkanal für die Dauerspülung und ist, wie die Fig. zeigt, von außen über einen mit Ventil versehenen Anschluß 5 erreichbar, an den ein Schlauch anschließbar ist. Ein weiterer gleichartiger Anschluß 6 zum Anschließen eines weiteren Schlauches ist an den Ringspalt zwischen Schaftrohr 2 und Außenrohr 4 angeschlossen, der als Rücklaufkanal dient.

Die beiden Rohre 2, 4 bestehen in üblicher Ausbildung aus Metall. Der distale Endabschnitt des Schaftrohres 2 ist in üblicher Weise isolierend, beispielsweise als Keramikendstück 7 ausgebildet.

Im Inneren des Schaftrohres 2 verläuft achsparallel eine Optik 8, die in der dargestellten Montagestellung mit Ihrem distalen Objektiv 9 den Arbeitsbereich vor dem Keramikendstück 7 betrachtet und die proximal den Hauptkörper 3 durchläuft. Sie durchläuft von dort weiter ein im Hauptkörper 3 befestigtes Führungsrohr 10 und endet jenseits von dessen proximalem Ende mit einem Okular 11, an dessen Stelle auch ein Kamera sitzen kann.

Auf dem Führungsrohr 10 ist mit einer Führungsbohrung 12 ein Schiebekörper 13 in axialer Richtung verschiebbar gelagert. Am proximalen Ende des Führungsrohres 10 ist ein Endstück 14 befestigt, demgegenüber der Schiebekörper 13 im dargestellten Ausführungsbeispiel mit der üblichen Blattfeder 15 federnd abgestützt ist. Am Endstück 14 ist ein Daumenring 16 angeordnet, während am Schiebestück 13 ein Fingergriff 17 angeordnet ist. Mit einer Hand des Operateurs kann mit dem Daumen im Daumenring 16 und mit dem Zeigefinger am Fingergriff 17 angefaßt und der Schiebekörper 13 axial bewegt werden. Alternativ kann anstelle der erläuterten "aktiven" Betätigung auch eine "passive" Betätigung vorgesehen sein, bei der die Blattfeder 15 zwischen Schiebekörper 13 und Hauptkörper 3 angeordnet ist und auch die Angriffstellen 16, 17 an diesen Teilen sitzen.

Im dargestellten Resektoskop ist auswechselbar eine HF-beaufschlagbare Elektrode 18 vorgesehen, die in üblicher Ausbildung für die Prostataresektion als rechtwinklig zur Achsrichtung erstreckte Drahtschlinge ausgebildet ist. Die Elektrode 18 wird von einem Elektrodenträger 19 getragen, der als Außenisolierung mit innerem Leiterdraht 20 ausgebildet ist. Der Elektrodenträger 19 ist in üblicher Ausbildung mit einer Hülse 21 längsverschiebbar auf der Optik 8 gelagert und durchläuft das Schaftrohr 2 bis zum Hauptkörper 3. Dort durchläuft er einen seitlich verschwenkten Durchgangskanal 22 mit Ringdichtung 23 zur Flüssigkeitsabdichtung und verläuft von dessen proximaler Mündung unter größerem Achsabstand wiederum parallel zur Achse weiter bis in eine Aufnahmebohrung 24 im Schiebekörper 13. Anstelle der Aufnahmebohrung 24 kann als alternative Ausbildung der Aufnahme für den Elektrodenträger 19 z.B. auch eine nach proximal konisch zulaufende Öffnung, ein zur Seite offener Schlitz oder dergleichen vorgesehen sein.

Im proximalen Endbereich weist der Elektrodenträger 19 einen sein Endstück ausbildenden Befestigungsabschnitt 25 auf, der ausreichend fest z.B. massiv aus Metall ausgebildet ist, um dort den Elektrodenträger sicher mechanisch befestigen zu können. Distal anschließend weist der Elektrodenträger 19 einen Kontaktabschnitt 26 auf, der mit einer elektrisch leitenden Außenfläche versehen ist, welche mit dem Leiterdraht 20 des Elektrodenträger 19 elektrisch leitend verbunden ist.

Die Aufnahmebohrung 24 weist ferner in Form ihres proximalen Endes 27 einen Endanschlag für den Elektrodenträger 19 auf, bis zu dem dieser in proximaler Richtung in die Aufnahmebohrung 24 einsteckbar ist.

Ist der Elektrodenträger 19 in der dargestellten Montagestellung bis zum Endanschlag 27 in die Aufnahmebohrung 24 des Schiebekörpers 13 eingesteckt, so liegt er mit seinem Kontaktabschnitt 26 in einer Ausnehmung 28 des Schiebekörpers 13, in dem der Kontaktabschnitt 26 von Außen frei zugänglich ist. Er kann dort beispielsweise mit dem dargestellten Klemmstecker 29 am Ende eines zu einem nicht dargestellten HF-Generators weiterführenden Kabels 30 kontaktiert werden.

Unmittelbar proximal neben der Ausnehmung 28 im Bereich des Befestigungsabschnittes 25 des Elektrodenträgers 19 ist im Schiebekörper 13 eine Befestigungseinrichtung vorgesehen, die im Ausführungsbeispiel eine Querbohrung 31 aufweist, welche beispielsweise mit Innengewinde zum Einschrauben einer Klemmschraube versehen sein kann. Alternativ kann die Befestigungseinrichtung auch anders ausgebildet sein z.B. mit einem Schieber, der in eine Nut faßt, als Rastverbindung oder dergleichen.

Die Ausnehmung 28 kann, wie dargestellt, allseitig freien Zugang zum Kontaktabschnitt 26 des Elektrodenträgers 19 bieten oder kann beispielsweise auch als nur von einer Seite zugangsvermittelnder Schacht ausgebildet sein, z.B. gemäß US 4,919,131.

Ist der Elektrodenträger 19 im Schiebekörper 13 ordnungsgemäß befestigt und kontaktiert, so kann durch die beschriebene Schiebebewegung des Schiebekörpers 13 der gesamte Elektrodenträger 19 mit der Elektrode 18 gegenüber dem Schaftrohr 2 längs verschoben werden. Unter Beobachtung durch die Optik 8 kann bei Hochfrequenzbeaufschlagung der Elektrode 18 mit dieser schneidend unter axialer Bewegung gearbeitet werden.

Zum Auswechseln der Elektrode 18 wird der Klemmstecker 29 abgenommen und die Befestigungseinrichtung (Querbohrung 31) gelöst. Dann kann der Elektrodenträger komplett aus dem Resektoskop 1 in distaler Richtung herausgezogen werden. In umgekehrter Weise kann eine neue Elektrode in proximaler Richtung bis zum Endanschlag 27 eingeschoben, mechanisch befestigt und kontaktiert werden. Dabei ist es möglich, zunächst den Elektrodenträger 19 an der Querbohrung 31 mechanisch zu befestigen und die ordnungsgemäße Funktion durch Hin-und Herschieben des Schiebekörpers 13 auszuprobieren, bevor die Kontaktierung mit dem Klemmstecker 29 erfolgt.

Im dargestellten Ausführungsbeispiel trägt der Elektrodenträger 19 eine Elektrode 18 in Form einer üblichen Resektoskopschlinge. Anstelle der Elektrode 18 können auch anders geformte Elektroden vorgesehen sein, wie beispielsweise Knopfelektroden, Stiftelektroden, Rollenelektroden oder Elektroden in Messerform, die bei HF-Beaufschlagung koagulierend, vaporisierend oder schneidend arbeiten.

Es können auch bipolare Elektroden verwendet werden, bei denen der Elektrodenträger 19 also zwei an die beiden Pole einer HF-Quelle anzuschließende Elektroden trägt. In diesem Fall sind im Inneren des isolierenden Elektrodenträgers 19 zwei Leiterdrähte 20 vorzusehen. Am proximalen Ende des Elektrodenträgers sind entsprechend anstelle des dargestellten einen Kontaktabschnittes 26 zwei Kontaktabschnitte vorzusehen, die z. B. mit einem Doppelstecker kontaktiert werden können.

Im dargestellten Ausführungsbeispiel ist als Endanschlag für das Einstecken des Elektrodenträgers 19 das Ende 27 der Aufnahmebohrung 24 vorgesehen. Ein Endanschlag kann jedoch auch an der Klemmvorrichtung selbst vorgesehen sein, beispielsweise an der in die Gewindebohrung 31 anzuschraubenden Klemmschraube. Falls als Klemmeinrichtung beispielsweise ein Schieber vorgesehen ist, der in eine Nut auf dem Befestigungsabschnitt 25 eingreift, so kann auch an diesem Schieber ein geeigneter Anschlag vorgesehen sein, der in geeigneter Weise z. B. mit einem entsprechenden Anschlag auf dem Befestigungsabschnitt 25 zusammen wirkt.

Im Ausführungsbeispiels ist dargestellt, das im Schiebekörper 13 der ordnungsgemäß montierte Elektrodenträger 19 mit seinem Kontaktabschnitt 26 in einer Ausnehmung 28 nach außen frei zugänglich angeordnet ist, um, wie dargestellt, mit einem von außen herangeführten Stecker 29 kontaktier zu werden. Es sind auch andere Kontaktierungseinrichtungen möglich, bei denen beispielsweise der Kontaktabschnitt 26 mit einem Kontakt berührt wird, der am Schiebekörper 13, z.B. in dessen Innerem, befestigt ist und von dem ein Kabel auf andere als dargestellte Weise weiterführend verläuft. In diesem Fall kann die Ausnehmung 28 entfallen.

## Patentansprüche

1. Urologisches Resektoskop (1) mit einem axial erstreckten Schaftrohr (2), das mit seinem proximalen Ende an einem Hauptkörper (3) befestigt ist, wobei proximal von diesem, gegenüber dem Hauptkörper (3) achsparallel verschiebbar ein Schiebekörper (13) gelagert ist, der eine Aufnahme (24), eine Befestigungseinrichtung (31), sowie eine Kontaktiereinrichtung (28, 29) aufweist, wobei in dem Resektoskop (1) eine HF-beaufschlagbare Elektrode (18) mit einem Elektrodenträger (19), der einen außen isolierten Leiterdraht (20) aufweist, axial bis über das distale Ende (7) des Schaftrohres (2) verschiebbar, lagerbar ist, wobei der Elektrodenträger (19) in Montagestellung das Schaftrohr (2) sowie den Hauptkörper (3) bis in die Aufnahme (24) durchlaufend mit der Befestigungseinrichtung (31) befestigbar und mit der Kontaktiereinrichtung (28, 29) kontaktierbar ist, **da** **durch gekennzeichnet, daß** die Befestigungseinrichtung (31) proximal von der Kontaktiereinrichtung (28, 29) am Schiebekörper (13) angeordnet ist.

2. Resektoskop nach Anspruch 1, **dadurch gekennzeichnet, daß** im Bereich der Befestigungseinrichtung (31) ein Endanschlag (27) für den Elektrodenträger (19) vorgesehen ist.

## Claims

1. Urological resectoscope (1) with an axially extended stem tube (2) which is fixed with its proximal end on a main body (3), wherein a slide member (13) is supported proximally thereof and displaceable in an axially parallel manner relative to the main body (3) and has a receptacle (24), a fixing device (31) as well as a contacting means (28, 29), wherein an electrode (18) which can be supplied with HF and has an electrode support (19) having an externally insulated conductor wire (20) can be supported in the resectoscope (1) so as to be axially displaceable beyond the distal end (7) of the stem tube (2), wherein in the assembled position the electrode support (19) running through the stem tube (2) and the main body (3) and into the receptacle (24) can be fixed with the fixing device (31) and can be contacted with the contacting means (28, 29), **characterised in that** the fixing device (31) is disposed proximally relative to the contacting means (28, 29) on the slide member (13).

2. Resectoscope as claimed in Claim 1, **characterised in that** an end stop (27) for the electrode support (19) is provided in the region of the fixing device (31).

## Revendications

1. Résectoscope urologique (1) muni d'une gaine (2) s'étendant dans le sens axial fixé par son extrémité proximale à un corps principal (3) du côté proximal duquel est logé un corps coulissant (13), lequel peut coulisser par rapport au corps principal (3) parallèlement à son axe et présente un logement (24), un dispositif de fixation (31) et un dispositif de contactage (28, 29), le résectoscope (1) pouvant recevoir une électrode (18) à laquelle peut être appliqué un courant HF et laquelle est tenue par un porte-électrode (19) doté d'un fil conducteur (20) isolé et peut être déplacée axialement jusqu'au-delà de l'extrémité distale (7) de la gaine (2), le porte-électrode (19) traversant la gaine (2) ainsi que le corps principal (3) jusque dans le logement (24) et pouvant, position de montage, être fixé par le dispositif de fixation (31) et contacté par le dispositif de contactage (28, 29), **caractérisé en ce que** le dispositif de fixation (31) est agencé dans le corps coulissant (13) en une position proximale par rapport au dispositif de contactage (28, 29).

2. Résectoscope selon la revendication 1, **caractérisé en ce qu'**une butée de fin de course (27) pour le porte-électrode (19) est prévue au niveau du dispositif de fixation (31).
